# EUROPEAN PATENT APPLICATION

(11) **EP 4 487 879 A1**
(43) Date of publication of application: **08.01.2025**
(21) Application number: 23784650.6
(22) Date of filing: 24.03.2023
(51) Int. Cl.: A61M 1/00

(54) **CONTAINMENT VESSEL AND WASTE LIQUID SUCTION DEVICE INCLUDING SAME**

(30) Priority: 08.04.2022 JP 2022064463
(71) Applicant: Daiken Medical Co., Ltd., Izumi-shi Osaka 594-1157 (JP)
(72) Inventor: YAMADA, Keiichi, Izumi-shi, Osaka 594-1157 (JP)
(74) Representative: TBK
(86) International application number: PCT/JP2023/011875
(87) International publication number: WO 2023/195360

(57) **Abstract**

A containment vessel (3) has a restricting containment portion (25) that has a higher rigidity than a restricted area (31) set in advance in a storage container (2) as an area that expands in accordance with formation of a negative pressure in the storage container (2) to restrict rupture, the restricting containment portion (25) containing the restricted area (31). The restricted area (31) has a flexibility capable of expanding from a contracted state (ST1) before formation of the negative pressure to a most expanded state (ST2) of being most expanded by the formation of the negative pressure, and the restricting containment portion (25) includes a restriction surface (29) having a shape along the restricted area (31) in an expansion process state (ST3) so as to be able to come into close contact with the entire restricted area (31) in the expansion process state (ST3) between the contracted state (ST1) and the most expanded state (ST2).

## Description

### Technical Field

The present invention relates to a waste liquid suction device that includes a storage container for storing a waste liquid and a containment vessel having a containment chamber for containing the storage container, and sucks the waste liquid by forming a negative pressure in a space outside the storage container in the containment chamber and inside the storage container.

### Background Art

Conventionally, for example, a suction device described in Patent Literature 1 has been known. This suction device includes a storage container (a storage bag) that stores body fluid and secretions of a patient, and a containment vessel (an attachment member, a main body, and a bottle) that contains the storage container.

The containment vessel has a supply hose to be connected to a supply source of a negative pressure. When the negative pressure is supplied through the supply hose, the negative pressure acts on an inside of the containment vessel, and a negative pressure acts on an inside of the storage container in accordance with the negative pressure. The body fluid and the secretions can be sucked into the storage container using this negative pressure.

The storage container is formed of a flexible material in order to reduce a volume before the waste liquid is sucked (before use) and secure a sufficient capacity for storing the waste liquid at the time of suction of the waste liquid.

Therefore, when a negative pressure is formed only in a space inside the storage container while an outside of the storage container is maintained at an atmospheric pressure, the storage container contracts, and the waste liquid cannot be effectively sucked into the storage container. Therefore, as described in Patent Literature 1, a negative pressure is formed not only in the space inside the storage container (hereinafter, referred to as a storage container inside space) but also in a space inside the containment vessel and outside the storage container (referred to as a storage container outside space).

However, when the negative pressure is formed in the two spaces as described above, it is difficult to simultaneously form the negative pressure in both the spaces. Therefore, when the negative pressure is formed in the storage container outside space before the storage container inside space in an initial stage of suction of the waste liquid, the storage container may expand more than necessary, and there is a risk that the storage container is ruptured. Therefore, it is necessary to give a strength to the storage container. As a result, it is difficult to manufacture the storage container using an inexpensive material because of a low strength.

### Citation List

### Patent Literature

Patent Literature 1: Japanese Unexamined Patent Publication No. 2020-32016

### Summary of Invention

An object of the present invention is to provide a containment vessel that can suppress rupture of a storage container even when the storage container made of an inexpensive material having a low strength is used, and a waste liquid suction device including the containment vessel.

### Means for Solving the Problem

A containment vessel according to one aspect of the present invention is a containment vessel in a waste liquid suction device that includes a storage container for storing a waste liquid and a containment vessel having a containment chamber for containing at least a part of the storage container, and sucks the waste liquid by forming a negative pressure in a space outside the storage container in the containment chamber and inside the storage container, the containment vessel having: a restricting containment portion that has a higher rigidity than a restricted area set in advance in the storage container as an area that expands in accordance with formation of the negative pressure in the storage container to restricts rupture, the restricting containment portion containing the restricted area, wherein the restricted area has a flexibility capable of expanding from a contracted state before the formation of the negative pressure to a most expanded state of being most expanded by the formation of the negative pressure, and the restricting containment portion includes a restriction surface having a shape along the restricted area in an expansion process state so as to be able to come into close contact with the entire restricted area in the expansion process state between the contracted state and the most expanded state.

In addition, a waste liquid suction device according to another aspect of the present invention includes: a storage container for storing a waste liquid; and a containment vessel having a containment chamber for containing at least a part of the storage container, wherein a negative pressure is formed in a space outside the storage container in the containment chamber and inside the storage container to suck the waste liquid.

According to the present invention, it is possible to provide a containment vessel that can suppress rupture of a storage container even when the storage container made of an inexpensive material having a low strength is used, and a waste liquid suction device including the containment vessel.

The objects, features, and advantages of the present invention will become more apparent from the following detailed description and the accompanying drawings.

### Brief Description of Drawings

FIG. 1 is a side cross-sectional view of a waste liquid suction device according to a first embodiment.
FIG. 2 is an enlarged side cross-sectional view illustrating a periphery of a passage forming member of the waste liquid suction device in FIG. 1.
FIG. 3 is a perspective view illustrating a state where a containment vessel is in a closed state in the waste liquid suction device of FIG. 1.
FIG. 4 is a perspective view illustrating a state where the containment vessel is in an open state and a storage container is attached in the waste liquid suction device of FIG. 1.
FIG. 5 is a front view illustrating a state where the storage container is removed in the waste liquid suction device illustrated in FIG. 4.
FIG. 6 is an enlarged perspective view illustrating an upper portion of the storage container in the waste liquid suction device of FIG. 1.
FIG. 7 is an enlarged front view illustrating the upper portion of the storage container in the waste liquid suction device of FIG. 1.
FIG. 8 is a plan view of the storage container in the waste liquid suction device of FIG. 1.
FIG. 9 is a view corresponding to FIG. 5 illustrating a modification of the waste liquid suction device according to the first embodiment.

### Description of Embodiments

Hereinafter, embodiments of the present invention will be described with reference to the accompanying drawings. Note that the following embodiments are examples embodying the present invention, and do not limit the technical scope of the present invention.

### (First Embodiment)

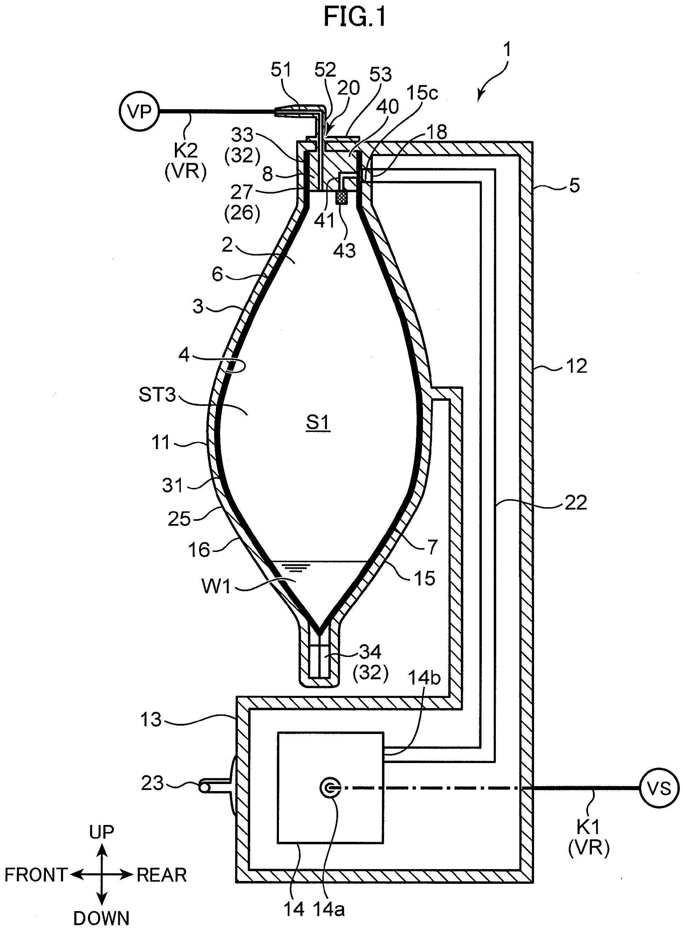

A waste liquid suction device 1 according to a first embodiment will be described with reference to FIGS. 1 to 5. The waste liquid suction device 1 according to the first embodiment is a device configured to suck a waste liquid W1 by a suction force of a predetermined suction source VS and store this sucked waste liquid W1. Specifically, the waste liquid suction device 1 is configured to suck the waste liquid W1 containing a body fluid (blood or the like) of a patient or a cleaning liquid (for example, physiological saline) for cleaning a body cavity generated during surgery or the like using the suction source VS disposed in a medical facility or the like, and store this sucked waste liquid W1.

The waste liquid suction device 1 is disposed between a suction starting point portion VP and the suction source VS on a suction path VR that is a suction path from the suction starting point portion VP serving as a suction starting point of the waste liquid W1 to the suction source VS. The suction path VR has a suction-side tube K1 connecting the suction source VS and the waste liquid suction device 1, and a patient-side tube K2 connecting the waste liquid suction device 1 and the suction starting point portion VP.

The waste liquid suction device 1 includes a storage container 2 for storing the waste liquid W1, and a main body portion 5 provided with a containment vessel 3 having a containment chamber 4 for storing at least a part of the storage container 2. The waste liquid suction device 1 is configured to suck the waste liquid W1 from the patient-side tube K2 into the storage container 2 by forming a negative pressure in a space outside the storage container 2 in the containment chamber 4 and inside the storage container 2.

The storage container 2 has a storage container body 6 that forms a storage space S1 for storing the waste liquid W1, and a passage forming member 8 for forming a suction passage (a part of the suction path VR) that communicates an inside and an outside of the storage container body 6.

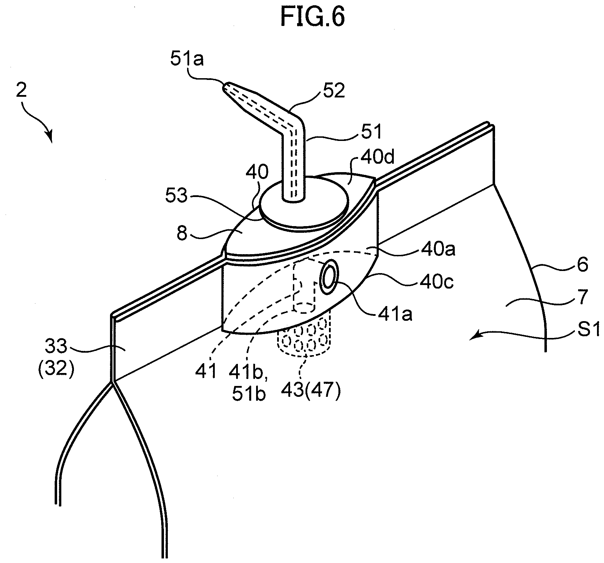

As illustrated in FIGS 6 to 8, the storage container body 6 has the storage space S1 formed therein by closing upper and lower opening portions of a tubular sheet member (a reference sign omitted). Specifically, the storage container body 6 has the tubular sheet member and bonding portions 32 that join sheet portions 7 facing each other in the tubular sheet member at upper and lower end portions of the tubular sheet member. The tubular sheet member has air impermeability and water impermeability, and is formed of, for example, a polyethylene resin.

Each of the bonding portions 32 is a portion formed by bonding sheet portions of the pair of sheet portions 7 of the tubular sheet member to each other by thermal welding, an adhesive, or the like. Specifically, the bonding portion 32 has a pair of upper bonding portions 33 that bonds upper portions of the pair of sheet portions 7 in an upper portion of the tubular member, and a lower bonding portion 34 that bonds the pair of sheet portions 7 in a lower portion of the storage container 2. The upper bonding portions 33 are formed by bonding the pair of sheet portions 7 adjacent to the passage forming member 8 described later. Note that in the present embodiment, the pair of upper bonding portions 33 is provided on both sides of the passage forming member 8.

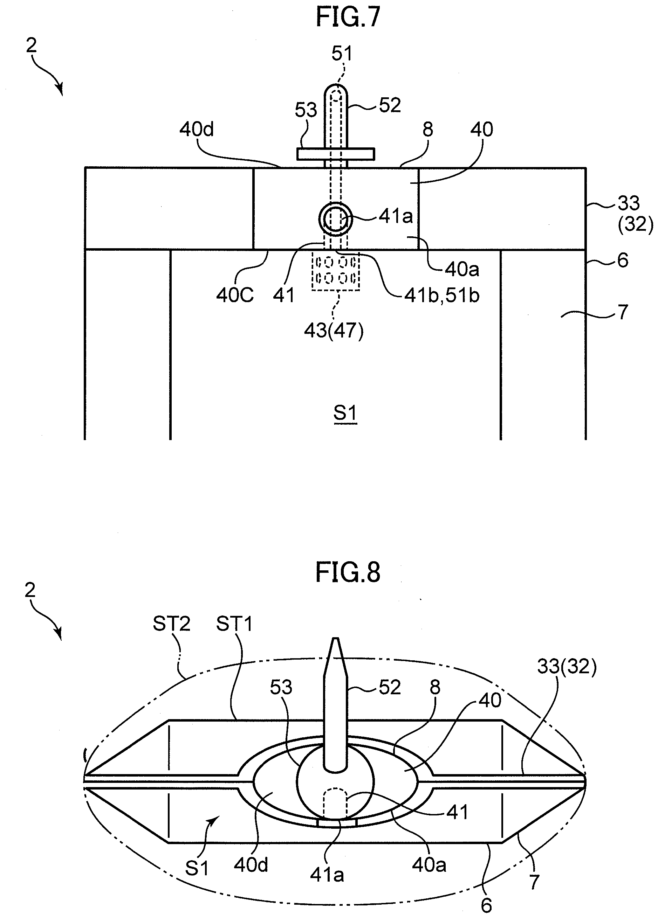

In addition, the storage container body 6 has a restricted area 31 set in advance in the storage container 2 (the tubular sheet member in the present embodiment) as an area that expands in accordance with formation of a prescribed negative pressure when the prescribed negative pressure (a maximum negative pressure allowed for the waste liquid suction device 1) is formed in the space outside the storage container 2 in the containment chamber 4 (hereinafter, referred to as a container outside space) and inside the storage container 2 (hereinafter, referred to as a container inside space). Specifically, the restricted area 31 is an area that comes into close contact with an inner surface of the containment vessel 3 described later at the time of forming the prescribed negative pressure, and is an area other than an area that faces a groove 21 of the containment vessel 3 described later. In addition, the restricted area 31 has a flexibility capable of expanding from a contracted state ST1 (a state indicated by a solid line in FIG. 8) before the formation of the prescribed negative pressure to a most expanded state ST2 (a state indicated by a two-dot chain line in FIG. 8) where the restricted area is most expanded by formation of the prescribed negative pressure. Note that the most expanded state ST2 is set in advance as a state where there is a risk that the storage container 2 is ruptured when the storage container 2 is further expanded beyond the most expanded state ST2. Specifically, since the storage container body 6 has the sheet portions 7 having a relatively low rigidity, when a pressure difference corresponding to the prescribed negative pressure occurs between the container outside space and the container inside space, the storage container body 6 expands from the contracted state ST1 in accordance with this pressure difference. Note that in the present embodiment, the storage container body 6 in the contracted state ST1 has an outer shape of a pillow shape in which a width (a length in a front-rear direction) gradually decreases from a center toward both ends in an up-down direction.

Note that as described above, the storage container body 6 (the restricted area 31) expands from the contracted state ST1. Therefore, a force acting on both the sheet portions 7 due to this expansion acts in a direction in which each of the bonding portions 32 (the sheet portions 7) in the storage container body 6 is peeled off. The containment vessel 3 described later is provided with means for restricting the peeling-off of the bonding portions 32 when the prescribed negative pressure is formed.

The passage forming member 8 is fixed to both the sheet portions 7 in a state of being sandwiched between the pair of sheet portions 7 facing each other in the storage container body 6 (an upper portion of the storage container body 6 in the present embodiment). Specifically, the passage forming member 8 has a sandwiched portion 40 sandwiched between the sheet portions 7, a connection pipe 52 provided on a side opposite to the storage container body 6 with respect to the sandwiched portion 40 and to be connected to the patient-side tube K2, a flange 53 extending from an outer peripheral surface of the connection pipe 52 at a position away from the sandwiched portion 40, and an air-permeable water-impermeable portion 43 connected to the sandwiched portion 40 inside the storage container body 6.

The sandwiched portion 40 is bonded (welded or be caused to adhere) to both the sheet portions 7 in the state of being sandwiched between the pair of sheet portions 7 facing each other in the upper bonding portions 33. Specifically, the sandwiched portion 40 has a planar cross-sectional shape tapered toward a boundary portion between both the sheet portions 7 in order to improve bonding strength to both the sheet portions 7. Both side surfaces 40a, 40b of the sandwiched portion 40 in the front-rear direction are bonded to the respective sheet members of the pair of sheet portions 7.

The connection pipe 52 has a base end portion extending from an upper surface 40d of the sandwiched portion 40 in a direction (upward) away from the storage container body 6, and a distal end portion extending perpendicularly to the base end portion from the base end portion and to be connected to the patient-side tube K2.

Further, the passage forming member 8 of the storage container 2 is formed with a first connection passage 41 for communicating the suction source VS (the containment vessel 3 described later) and the storage space S1, and a second connection passage 51 for communicating the storage space S1 and the patient-side tube K2. The first connection passage 41 is a passage that connects an inside and an outside of the storage container 2. In the present embodiment, as illustrated in FIG. 6, the first connection passage 41 is formed in the storage container body 6 and the sandwiched portion 40 so as to connect an opening 41a and an opening 41b, the opening 41a being opened in a direction in which the side surface 40a of the sandwiched portion 40 faces through a hole formed in the sheet portion 7, and the opening 41b being formed on a lower surface of the sandwiched portion 40. Here, the opening 41b formed in a lower surface 40c of the sandwicheds portion 40 in the first connection passage 41 is closed by the air-permeable water-impermeable portion 43 having air permeability and water impermeability. In addition, the second connection passage 51 is provided across the sandwiched portion 40 and the connection pipe 52 so as to connect an opening 51a and an opening 51b, the opening 51a being formed at the distal end portion of the connection pipe 52, and the opening 51b being formed in the sandwiched portion 40 and opened inside the storage container body 6.

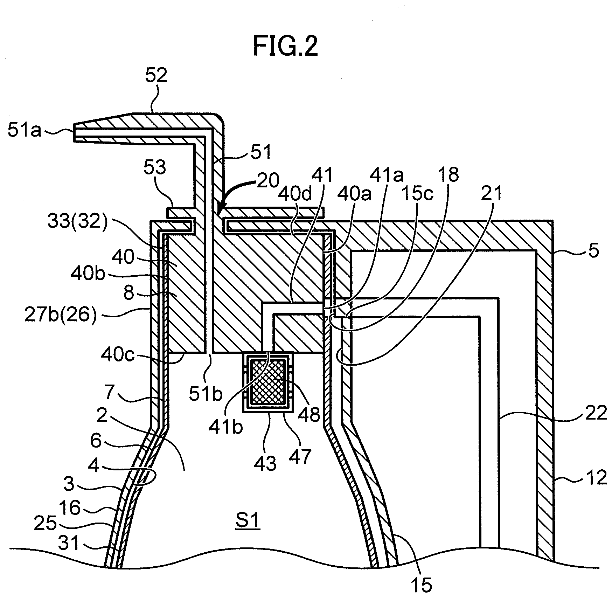

The air-permeable water-impermeable portion 43 is to allows gas to flow through the first connection passage 41 and to regulate liquid flow through the first connection passage 41. Specifically, as illustrated in FIG. 2, the air-permeable water-impermeable portion 43 has a water stop filter 48 attached to the lower surface 40c of the sandwiched portion 40 so as to cover the opening 41b of the first connection passage 41, and a filter case 47 attached to the sandwiched portion 40 so as to cover the water stop filter 48. The water stop filter 48 is formed of, for example, a porous polyethylene sheet having a plurality of pores, a microporous polypropylene resin, or a material having air permeability and water impermeability such as a nonwoven fabric.

The flange 53 cooperates with the sandwiched portion 40 to position the storage container 2 with respect to the containment vessel 3 described later. Specifically, as illustrated in FIG. 2, the storage container 2 is contained inside the containment vessel 3 described later in a state where a portion of the connection pipe 52 between the flange 53 and the sandwiched portion 40 is sandwiched by the containment vessel 3. In this contained state, since a part of the containment vessel 3 is disposed between the flange 53 and the sandwiched portion 40, the storage container 2 can be positioned in the up-down direction with respect to the containment vessel 3.

Next, the main body portion 5 will be described. The main body portion 5 has the containment vessel 3 having the containment chamber 4 for containing a part of the storage container 2, an operated portion 13 having dials 23 for adjusting the negative pressure to be formed inside the storage container 2, and a joining portion 12 for joining the containment vessel 3 and the operated portion 13.

The operated portion 13 has a regulator 14 for adjusting the suction force from the suction source VS, a housing portion (a reference sign is omitted) for housing the regulator, and the dials 23 connected to the regulator 14 in a state of being exposed to an outside (a front surface) of the housing portion. The regulator 14 includes a connection port 14a to which the suction-side tube K1 is to be connected and a connection port 14b to which suction piping 22 described later is connected. The dials 23 are configured to adjust a supply amount of the negative pressure in the regulator 14 and switch on and off the negative pressure supply.

The joining portion 12 has a leg portion extending upward from the operated portion 13 and a connection portion extending forward from the leg portion and connected to the containment vessel 3. In addition, the joining portion 12 communicates with a space inside the housing portion of the operated portion 13, and has an internal space in which the suction piping 22 described later is disposed. Note that the internal space of the joining portion 12 is closed from a front side by a containment member 15 of the containment vessel 3 described later.

The containment vessel 3 has the containment chamber 4 configured to contain the entire storage container body 6. The containment vessel 3 has a pair of containment members 15, 16 that are detachably connected to each other in a facing direction of the pair of sheet portions 7 so as to expose a part of the passage forming member 8 (the connection pipe 52) and sandwich the passage forming member 8, thereby containing the entire storage container body 6 of the storage container 2.

The pair of containment members 15, 16 is disposed side by side in the front-rear direction. Of the containment members 15, 16, the containment member 15 disposed on a rear side is connected to the joining portion 12 as described above. In addition, the containment member 15 has a suction opening portion 18 that opens into the containment chamber 4, and a communication passage 15c that communicates the internal space with the containment chamber 4 is formed. One end of the suction piping 22 is connected to the communication passage 15c, and another end of the suction piping 22 is connected to the connection port 14b of the regulator 14. The suction opening portion 18 is opened on a front surface of the containment member 15 in upper sandwiching portions 27a described later of the containment vessel 3. Specifically, the suction opening portion 18 is formed at a position facing the opening 41a in the storage container body 6 contained inside the containment chamber 4.

The containment chamber 4 is connected to the regulator 14 and the suction-side tube K1 by the suction piping 22. That is, the containment vessel 3 has the suction opening portion 18 that opens into the containment chamber 4, and the suction passage (the communication passage 15c, the suction piping 22, and the regulator 14) connected to the suction source VS can be connected to the suction opening portion 18. Further, this suction passage communicates with the storage space S1 through the opening 41a of the storage container body 6, and is to be connected to the patient-side tube K2 via the passage forming member 8. As a result, the storage space S1 of the storage container 2 and the suction starting point portion VP communicate with each other through the second connection passage 51. Furthermore, the suction path VR, which is a suction path from the suction source VS to the suction starting point portion VP, is formed in the waste liquid suction device 1 by the first connection passage 41, the second connection passage 51, the suction piping 22, and the regulator 14.

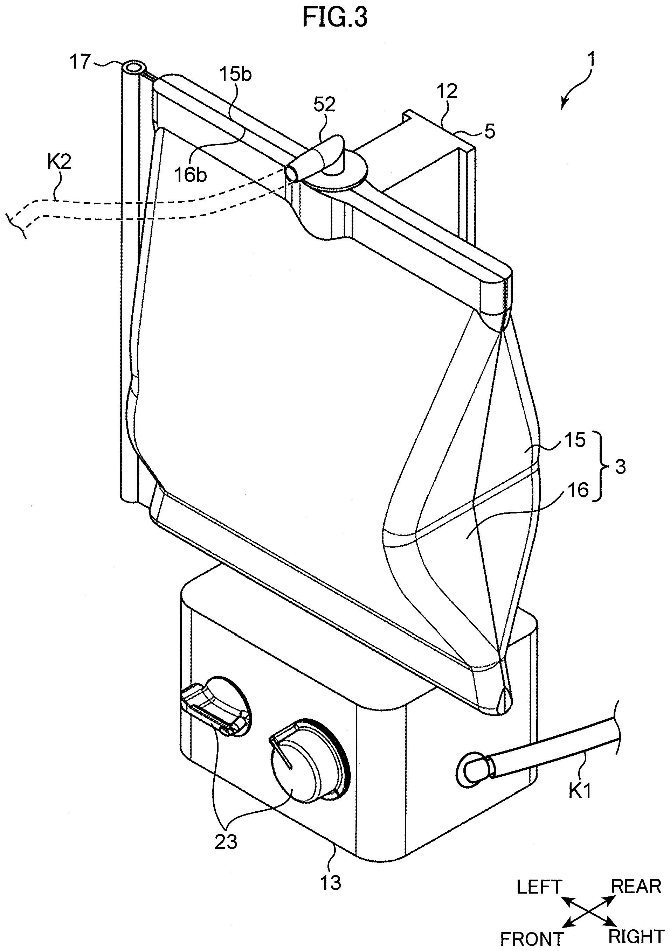

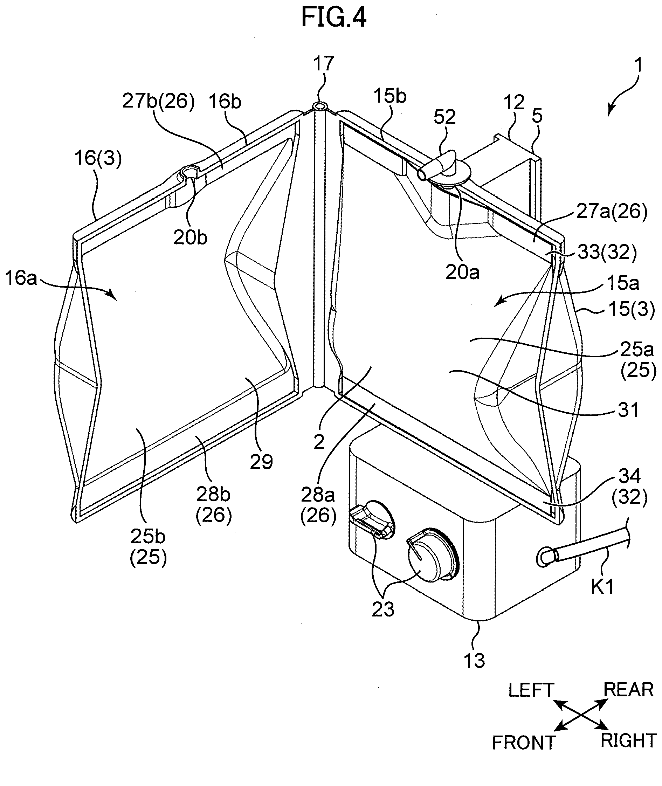

In addition, the pair of containment members 15, 16 is connected to each other by a hinge 17 disposed at one end in a width direction (a left end in FIG. 3) so as to be rotatable about an axis extending in the up-down direction as a rotation axis. Specifically, the containment member 16 is rotatable between a closed state illustrated in FIG. 3 for containing the storage container 2 and an open state illustrated in FIG. 4 for opening the storage container 2 forward. In an upper portion of the containment vessel 3 in the closed state, a through hole 20 that communicates the containment chamber 4 with the outside is formed in order to insert the connection pipe 52 of the storage container 2 from the inside (the containment chamber 4) to the outside of the containment members 15, 16. The through hole 20 is formed across the pair of containment members 15, 16. That is, the through hole 20 is configured of a notch 20a that opens forward on a front surface of an upper portion of the containment member 15 and a notch 20b that opens rearward on a rear surface of an upper portion of the containment member 16. When the containment vessel 3 is brought into the closed state, the notch 20a of the containment member 15 and the notch 20b of the containment member 16 overlap each other, and the through hole 20 is formed in the containment vessel 3.

The containment member 15 has a first recessed portion 15a that opens to a front side of the containment member 15 and forms a rear portion of the containment chamber 4. That is, the containment member 15 has a rear wall extending in a left-right direction and the up-down direction, and a frame portion 15b extending forward from a peripheral edge of the rear wall, and the first recessed portion 15a is defined by the rear wall and the frame portion 15b.

Similarly, the containment member 16 opens to a rear side of the containment member 16, and a second recessed portion 16a forming a front portion in the containment chamber 4 is formed. That is, the containment member 16 has a front wall extending in the left-right direction and the up-down direction in the closed state, and a frame portion 16b extending rearward from a peripheral edge of the front wall, and the second recessed portion 16a is defined by the front wall and the frame portion 16b.

In the containment vessel 3, the containment member 16 is rotated to the containment member 15 such that the frame portion 16b and the frame portion 15b are in close contact with each other and the frame portions 15b, 16b and the storage container 2 (the connection pipe 52) are in close contact with each other, whereby the containment vessel 3 is brought into the closed state where the containment chamber 4 is formed. In the containment vessel 3 in the closed state, airtightness of the containment chamber 4 is maintained by restraining the containment member 15 and the containment member 16 to each other by restraining means (not illustrated). Note that a member (packing or the like) for ensuring airtightness between the frame portion 15b and the frame portion 16b and between both the frame portions 15b, 16b and the storage container 2 (the connection pipe 52) can also be provided. On the other hand, in the containment vessel 3, by separating the containment member 16 from the containment member 15, the containment vessel 3 is brought into the open state where the first recessed portion 15a and the second recessed portion 16a, that is, the storage container 2 is opened to the outside. In the containment vessel 3 in the open state, the storage container 2 can be taken into and out of the containment chamber 4.

Each of the containment members 15, 16 has a restricting containment portion 25 that has a higher rigidity than the restricted area 31 and contains the restricted area 31 of the storage container 2, and a sandwiching portion 26 for sandwiching the bonding portions 32 of the storage container 2 in the facing direction (the front-rear direction) of the pair of sheet portions 7.

The sandwiching portion 26 has the pair of upper sandwiching portions 27a provided in the containment member 15 to sandwich the upper bonding portions 33, a pair of upper sandwiching portions 27b provided in the containment member 16 to sandwich the upper bonding portions 33, a lower sandwiching portion 28a provided in the containment member 15 to sandwich the lower bonding portion 34, and a lower sandwiching portion 28b provided in the containment member 16 to sandwich the lower bonding portions 34. The upper sandwiching portions 27a, 27b sandwich, in the front-rear direction, the upper bonding portions 33 of the storage container 2 in the containment vessel 3 in the closed state. The lower sandwiching portions 28a, 28b sandwich, in the front-rear direction, the lower bonding portion 34 of the storage container 2 in the containment vessel 3 in the closed state. Further, each of the containment members 15, 16 has another sandwiching portion (a reference sign is omitted) for sandwiching the sandwiched portion 40. The other sandwiching portion is provided between the pair of upper sandwiching portions 27a in the containment member 15, and is provided between the pair of upper sandwiching portions 27b in the containment member 16.

The restricting containment portion 25 includes a restricting containment portion 25a provided in the containment member 15 and a restricting containment portion 25b provided in the containment member 16 between the upper sandwiching portions 27a, 27b (the other sandwiching portions) and the lower sandwiching portions 28a, 28b. In addition, the restricting containment portions 25a, 25b are provided at positions facing the restricted area 31 of the storage container 2. The restricting containment portions 25a, 25b include a restriction surface 29 having a shape along the restricted area 31 in an expansion process state ST3 so that the restriction surface 29 can come into close contact with the entire restricted area 31 in the expansion process state ST3 (a state illustrated in FIG. 1) between the contracted state ST1 (the state indicated by solid line in FIG. 8) and the most expanded state ST2 (the state indicated by two-dot chain line in FIG. 8). Note that "between the contracted state ST1 and the most expanded state ST2" is intended to include the contracted state ST1 and the most expanded state ST2. In the present embodiment, since the storage container body 6 is molded so that the storage space S1 is formed in the contracted state ST1, the restriction surface 29 for restricting the expansion of the storage container 2 in the contracted state ST1 is exemplified.

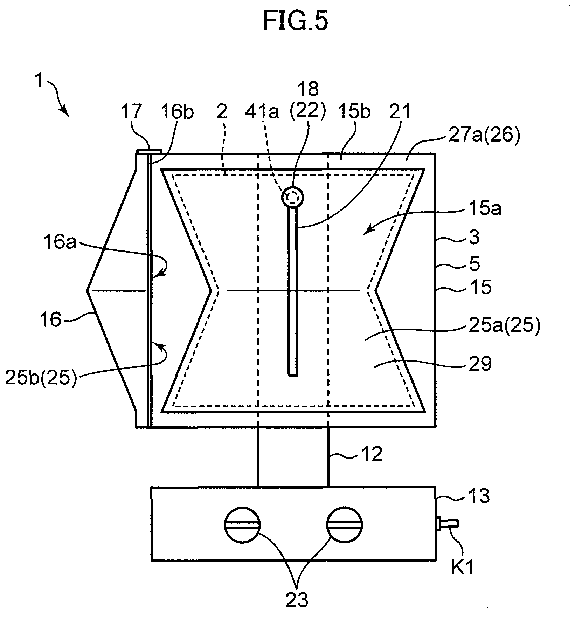

As illustrated in FIG. 5, the containment member 15 includes a groove 21 that extends from the suction opening portion 18 along an inner surface of the containment vessel 3 facing a side of the containment chamber 4 and opens toward the side of the containment chamber 4. The groove 21 is formed on the inner surface of the containment member 15 so as to extend downward from the suction opening portion 18 toward a lower portion of the restriction surface 29 of the restricting containment portion 25. As a result, even in a state where the storage container body 6 is in close contact with the inner surface of the containment member 15, the negative pressure acting on the upper portion of the containment chamber 4 through the suction opening portion 18 can be developed to a down portion of the containment vessel 3. Note that the restriction surface 29 is an inner surface of the restricting containment portions 25a, 25b that are in close contact with the storage container body 6 in a state where the prescribed negative pressure acts on the waste liquid suction device 1, and is a portion excluding an area facing the groove 21.

The containment vessel 3 (the containment members 15, 16) has a higher rigidity than the restricted area 31 of the storage container 2. Therefore, when the prescribed negative pressure acts on the waste liquid suction device 1, the storage container body 6 having a rigidity lower than that of the containment vessel 3 is deformed without deforming the containment vessel 3, so that a volume of the storage space S1 changes. Specifically, in a state where the prescribed negative pressure does not act on the waste liquid suction device 1, the storage container body 6 is in the contracted state ST1. On the other hand, in a case where the prescribed negative pressure acts on the waste liquid suction device 1 without restricting the expansion by the restriction surface 29, and a differential pressure is generated between the container outside space and the container inside space, the storage container body 6 is brought into the most expanded state ST2, and there is a risk that the storage container 2 is ruptured when further expansion occurs. As described above, since the restriction surface 29 is provided in the containment vessel 3, the expansion of the storage container 2 is restricted in the expansion process state, and the storage container 2 can be prevented from expanding beyond the most expanded state ST2, so that the rupture of the storage container 2 can be suppressed.

Note that the containment vessel 3 and the storage container body 6 are preferably formed of a colorless and transparent synthetic resin having optical transparency, for example, so that a storage state of the waste liquid W1 inside the storage container 2 contained in the containment chamber 4 can be visually recognized. Note that the containment vessel 3 and the storage container body 6 may be formed of a colored synthetic resin or metal.

### (Operation and Effects)

According to the waste liquid suction device 1 configured above, since the restricting containment portion 25 having a higher rigidity than the restricted area 31 has the restriction surface 29 capable of being in close contact with the entire restricted area 31 in the expansion process state before expansion up to the most expanded state ST2, the restricted area 31 can be prevented from being expanded more than necessary. As a result, even when a storage container made of an inexpensive material having a lower strength is used, it is possible to suppress rupture of the storage container 2.

In addition, in a case where the containment vessel 3 has the suction piping 22 that opens into the containment chamber 4 and is connected to the suction source VS, the expanded storage container 2 closes the opening, so that there is a risk that the negative pressure cannot be effectively formed in the space outside the storage container 2. However, in the waste liquid suction device 1, even when the suction opening portion 18 of the suction piping 22 (the suction passage) is closed by the storage container 2, a formation range of the negative pressure can be developed along the inner surface of the containment vessel 3 through the groove 21.

In addition, the storage container 2 has the first connection passage 41 (the connection passage) that connects the inside and the outside of the storage container 2, and the air-permeable water-impermeable portion 43 that is provided in the first connection passage 41 so as to close the first connection passage 41 and has air permeability and water impermeability, and the storage container 2 can be contained in the containment vessel 3 in a state where the air-permeable water-impermeable portion 43 is disposed inside the containment chamber 4. In this way, the negative pressure acting on the space outside the storage container 2 through the first connection passage 41 can also act on the inside of the storage container 2 through the air-permeable water-impermeable portion 43.

In addition, the storage container 2 is adopted that has the passage forming member 8 fixed to the sheet portions 7 in the state of being sandwiched by the pair of sheet portions 7. As a result, for example, by welding the sheet portions 7 and the passage forming member 8 in the state where the passage forming member 8 is sandwiched between the pair of sheet portions 7, the storage container 2 having the passage through the inside and outside of the storage container body 6 can be relatively easily manufactured.

Furthermore, by detachably attaching the pair of containment members 15, 16 in the facing direction of the pair of sheet portions 7 so as to sandwich the passage forming member 8, the entire storage container body 6 can be detachably contained in the state where a part of the passage forming member 8 is exposed in order to suck the waste liquid W1.

In addition, when the storage container 2 is expanded in accordance with the negative pressure, there is a risk that the storage container 2 is ruptured by receiving a force in a direction in which the bonding portions 32 are peeled off. However, in the waste liquid suction device 1, since the storage container body 6 can be contained in the containment vessel 3 in the state where the bonding portions 32 are sandwiched by the sandwiching portions 26 of the pair of containment members 15, 16, it is possible to suppress the peeling-off of the bonding portions 32 at the time of the expansion of the storage container 2 in accordance with the negative pressure.

### (Modification of First Embodiment)

In the waste liquid suction device 1 described above, the suction opening portion 18 of the main body portion 5 is disposed at the position facing the opening 41a of the first connection passage 41, but a positional relationship between the suction opening portion 18 and the opening 41a is not limited thereto. For example, the suction opening portion 18 may be provided at a position deviated from the opening 41a (the connection opening) in the storage container 2 that is contained in the containment vessel 3 and is in the expansion process state. In this case, it is preferable that the groove 21 formed in the containment member 15 extends along the inner surface of the containment vessel 3 so that the suction opening portion 18 and the opening 41a can be connected.

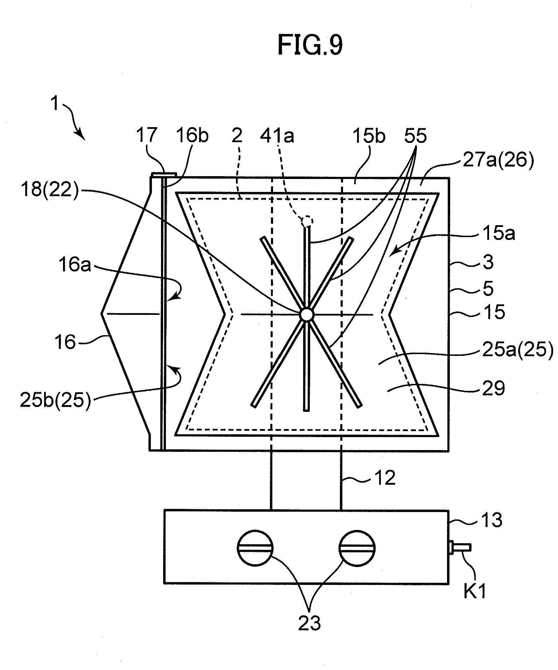

Specifically, in the modification illustrated in FIG. 9, the suction opening portion 18 is opened at a center of the inner surface of the containment member 15. In addition, the containment vessel 3 includes a plurality of grooves 55 extending from the suction opening portion 18 along the inner surface of the containment member 15. Among the plurality of grooves 55, the grooves 55 extending upward from the suction opening portion 18 allow the suction opening portion 18 and the opening 41a of the first connection passage 41 to communicate with each other in the state where the storage container 2 is contained in the containment chamber 4.

When the storage container 2 expanded up to the expansion process state comes into close contact with the inner surface of the containment vessel 3, thereby closing the opening 41 a (the opening portion) of the first connection passage 41, there is a risk that the negative pressure from the suction opening portion 18 cannot act on the inside of the storage container 2. However, in the waste liquid suction device 1 configured as described above, since the suction opening portion 18 and the first connection passage 41 can be connected by the grooves 55 even in a state where the storage container 2 expanded to the expansion process state comes into close contact with the inner surface of the containment vessel 3, the negative pressure acting on the space outside the storage container 2 through the suction opening portion 18 can reliably act on the inside of the storage container 2.

Note that the present invention is not limited to the above embodiments, and for example, aspects below can also be adopted.

In the above example, the containment vessel 3 capable of containing the entire storage container body 6 has been described, but the containment vessel 3 may be configured to contain only a part of the storage container body 6. In this case, for example, as described in Japanese Patent No. 4638347, a portion of the storage container 2 disposed outside the containment vessel 3 is required to have a rigidity to such an extent that the portion is not contracted by the negative pressure acting into the storage container 2.

In the above example, the first recessed portion 15a of the containment member 15 and the second recessed portion 16a of the containment member 16 are configured to divide the containment chamber 4 in half, but a ratio of the division may be changed to a ratio other than half.

Note that the above-described specific embodiments mainly include inventions having the following configurations.

A containment vessel according to one aspect of the present invention is a containment vessel in a waste liquid suction device that includes a storage container for storing a waste liquid and a containment vessel having a containment chamber for containing at least a part of the storage container, and sucks the waste liquid by forming a negative pressure in a space outside the storage container in the containment chamber and inside the storage container, the containment vessel having: a restricting containment portion that has a higher rigidity than a restricted area set in advance in the storage container as an area that expands in accordance with formation of the negative pressure in the storage container to restricts rupture, the restricting containment portion containing the restricted area, wherein the restricted area has a flexibility capable of expanding from a contracted state before the formation of the negative pressure to a most expanded state of being most expanded by the formation of the negative pressure, and the restricting containment portion includes a restriction surface having a shape along the restricted area in an expansion process state so as to be able to come into close contact with the entire restricted area in the expansion process state between the contracted state and the most expanded state.

According to the present invention, since the restricting containment portion having a higher rigidity than the restricted area has the restriction surface that can be brought into close contact with the entire restricted area in the expansion process state before being expanded to the most expanded state, it is possible to prevent the restricted area from expanding more than necessary.

Therefore, according to the present invention, it is possible to suppress rupture of the storage container even when the storage container made of an inexpensive material having a low strength is used.

Here, in a case where the containment vessel has a suction passage that opens into the containment chamber and is connected to a suction source, the expanded storage container closes the opening, and thus, there is a risk that the negative pressure cannot be effectively formed in the storage container outside space.

Consequently, it is preferable that the containment vessel includes: a suction opening portion that opens into the containment chamber and is configured to connect to a suction passage to be connected to a suction source; and a groove that extends from the suction opening portion along an inner surface of the containment vessel facing the containment chamber and opens toward the containment chamber.

According to this aspect, even when the suction opening portion of the suction passage is closed by the storage container, a formation range of the negative pressure can be developed along the inner surface of the containment vessel through the groove.

Here, the storage container has a connection passage connecting the inside and the outside of the storage container, and an air-permeable water-impermeable portion provided in the connection passage so as to close the connection passage and having air permeability and water impermeability, and the storage container can be stored in the containment vessel in a state where the air-permeable water-impermeable portion is disposed inside the containment chamber. In this way, the negative pressure acting on the container outside space through the suction passage can also act on the inside of the storage container through the air-permeable water-impermeable portion. However, when the storage container expanded to the expansion process state comes into close contact with the inner surface of the containment vessel, thereby closing the opening portion of the connection passage, there is a risk that the negative pressure from the suction opening portion cannot act on the inside of the storage container.

Therefore, in the containment vessel, it is preferable that the suction opening portion is provided at a position deviated from a connection opening of the connection passage opened to a side of the containment chamber in the storage container contained in the containment vessel and being in the expansion process state, and the groove extends along the inner surface of the containment vessel so as to be able to connect the suction opening portion and the connection opening.

According to this aspect, since the suction opening portion and the connection passage can be connected by the groove even in the state where the storage container expanded to the expansion process state comes into close contact with the inner surface of the containment vessel, the negative pressure acting on the container outside space through the suction opening portion can be reliably acted on the inside of the storage container.

In the containment vessel, it is preferable that the storage container has a storage container body for storing the waste liquid, and a passage forming member fixed to both sheet portions in a state of being sandwiched between the pair of sheet portions facing each other in the storage container body in order to form a suction passage through an inside and an outside of the storage container body, and that the containment vessel has a pair of containment members that is detachably connected to each other in a facing direction of the pair of sheet portions so as to expose a part of the passage forming member and sandwich the passage forming member, thereby containing the entire storage container body.

In this aspect, the storage container having the passage forming member fixed to the sheet portions in the state of being sandwiched by the pair of sheet portions is adopted. As a result, for example, by welding the sheet portions and the passage forming member in the state where the passage forming member is sandwiched between the pair of sheet portions, it is possible to relatively easily manufacture the storage container having the passage through the inside and outside of the storage container body.

Further, by detachably attaching the pair of containment members in the facing direction of the pair of sheet portions so as to sandwich the passage forming member, the entire storage container body can be detachably contained in a state where a part of the passage forming member is exposed in order to suck the waste liquid.

Here, the storage container body may have a bonding portion in which the pair of sheet portions is bonded adjacent to the passage forming member. In this case, when the storage container is expanded in accordance with the negative pressure, the storage container may be ruptured by receiving a force in a direction in which the bonding portion is peeled off.

Therefore, in the containment vessel, it is preferable that each of the pair of containment members has a sandwiching portion for sandwiching the bonding portion of the storage container in the facing direction of the pair of sheet portions.

According to this aspect, since the storage container body can be contained in the containment vessel in the state where the bonding portion is sandwiched by the sandwiching portions of the pair of containment members, it is possible to suppress peeling-off of the bonding portion at the time of expansion of the storage container in accordance with the negative pressure.

In addition, a waste liquid suction device according to another aspect of the present invention includes: a storage container for storing a waste liquid; and a containment vessel having a containment chamber for containing at least a part of the storage container, wherein a negative pressure is formed in a space outside the storage container in the containment chamber and inside the storage container to suck the waste liquid.

As described above, according to the present invention, it is possible to provide a containment vessel that can suppress rupture of a storage container even when the storage container made of an inexpensive material having a low strength is used, and a waste liquid suction device including the containment vessel.

## Claims

1. containment vessel in a waste liquid suction device that includes a storage container for storing a waste liquid and a containment vessel having a containment chamber for containing at least a part of the storage container, and sucks the waste liquid by forming a negative pressure in a space outside the storage container in the containment chamber and inside the storage container, the containment vessel having:
a restricting containment portion that has a higher rigidity than a restricted area set in advance in the storage container as an area that expands in accordance with formation of the negative pressure in the storage container to restrict rupture, the restricting containment portion containing the restricted area, wherein
the restricted area has a flexibility capable of expanding from a contracted state before the formation of the negative pressure to a most expanded state of being most expanded by the formation of the negative pressure, and
the restricting containment portion includes a restriction surface having a shape along the restricted area in an expansion process state so as to be able to come into close contact with the entire restricted area in the expansion process state between the contracted state and the most expanded state.

2. The containment vessel according to claim 1, comprising:
a suction opening portion that opens into the containment chamber and is configured to connect to a suction passage to be connected to a suction source; and
a groove that extends from the suction opening portion along an inner surface of the containment vessel facing the containment chamber and opens toward the containment chamber.

3. The containment vessel according to claim 2, wherein
the storage container has a connection passage that connects an inside and an outside of the storage container, and an air-permeable water-impermeable portion that is provided in the connection passage so as to close the connection passage and has air permeability and water impermeability,
the suction opening portion is provided at a position deviated from a connection opening of the connection passage opened to a side of the containment chamber in the storage container contained in the containment vessel and being in the expansion process state, and
the groove extends along the inner surface of the containment vessel so as to be able to connect the suction opening portion and the connection opening.

4. The containment vessel according to any one of claims 1 to 3, wherein
the storage container has a storage container body for storing the waste liquid, and a passage forming member fixed to both sheet portions in a state of being sandwiched between the pair of sheet portions facing each other in the storage container body in order to form a suction passage through the inside and the outside of the storage container body, and
the containment vessel has a pair of containment members that is detachably connected to each other in a facing direction of the pair of sheet portions so as to expose a part of the passage forming member and sandwich the passage forming member, thereby containing the entire storage container body.

5. The containment vessel according to claim 4, wherein
the storage container body has a bonding portion where the pair of sheet portions is bonded to each other adjacent to the passage forming member, and
each of the pair of containment members has a sandwiching portion for sandwiching the bonding portion of the storage container in the facing direction of the pair of sheet portions.

6. waste liquid suction device comprising:
a storage container for storing a waste liquid; and
the containment vessel according to claim 1 having the containment chamber for containing at least a part of the storage container, wherein
a negative pressure is formed in a space outside the storage container in the containment chamber and inside the storage container to suck the waste liquid.
